# EUROPÄISCHE PATENTSCHRIFT

(11) **EP 0 828 480 B1**
(45) Veröffentlichungstag und Bekanntmachung des Hinweises auf die Patenterteilung: **29.08.2001**
(21) Anmeldenummer: 96914833.7
(22) Anmeldetag: 24.05.1996
(51) Int. Cl.: A61K 9/16, A61K 9/50

(54) **NEUE PHARMAZEUTISCHE PELLETFORMULIERUNG**
NOVEL PHARMACEUTICAL PELLET FORMULATION
NOUVELLE FORMULATION PHARMACEUTIQUE DE PELLETS

(30) Priorität: 24.05.1995 CH 154295
(43) Veröffentlichungstag der Anmeldung: 18.03.1998
(73) Patentinhaber: Mepha AG, CH-4147 Aesch (CH)
(72) Erfinder: SCHEIWE, Max, Werner, D-79689 Maulburg (DE); VILLIGER, Thomas, CH-4132 Muttenz (CH)
(74) Vertreter: Braun, André
(86) Internationale Anmeldenummer: CH9600203
(87) Internationale Veröffentlichungsnummer: WO9637195

(56) Entgegenhaltungen:
- EP-A- 0 526 862

## Beschreibung

Die Erfindung betrifft eine neue stabile pharmazeutische Pelletformulierung enthaltend Omeprazol und ein Verfahren zur Herstellung dieser Formulierung.

Die pharmazeutischen Wirkungen von Omeprazol auf den Organismus von Lebewesen sind weitgehend erforscht und bekannt. Hingegen bereitete es bisher Mühe, pharmazeutische Formulierungen, die Omeprazol enthalten, über längere Zeit stabil zu halten. Da die Stabilität von Omeprazol von organischen Lösungsmitteln und Feuchtigkeit beeinflusst und dessen Umwandlung von sauer reagierenden Reagentien gefördert resp. von alkalisch reagierenden Reagentien verhindert wird, muss eine oral verabreichbare Omeprazolformulierung mit einem magensaftresistenten Überzug gegen die Wirkung der Magensäure geschützt werden, damit sie im Dünndarm ihre Wirkung entfalten kann.

Üblicherweise enthalten aber magensaftresistente Überzüge sauer reagierende Komponenten, so dass Omeprazol bei Kontaktnahme damit kontinuierlich zersetzt und damit im Laufe der Zeit sowohl sein Aussehen verändern als auch seine Wirkung verlieren würde.

Um diese Nachteile zu vermindern, wurde z.B. versucht, pharmazeutische Formulierungen mit Omeprazol mit zwei Überzügen zu versehen, wobei der innere Überzug eine Barriere gegen den äusseren das Omeprazol zersetzenden magensaftresistenten Überzug und gegen eindringende Feuchtigkeit bilden sollte.

Es hat sich aber gezeigt, dass die Stabilität solcher pharmazeutischen Formulierungen den gewünschten Kriterien nach wie vor nicht genügt, und dass das doppelte Überziehen der Formulierung das Herstellungsverfahren stark verteuert.

Aufgabe der vorliegenden Erfindung ist es deshalb, eine stabile pharmazeutische Formulierung mit einem Kern enthaltend Omeprazol als Wirkstoff und einem einzigen Überzug zur Verfügung zu stellen, wobei die oben erwähnten Nachteile vermieden werden sollen.

In der EP-A-526 862 sind Darreichungsformen für pharmazeutisch wirksame Verbindungen beschrieben, welche eine erheblich verlängerte Verweilzeit im Magen aufweisen. Dies wird dadurch erreicht, dass die pharmazeutisch aktive Verbindung mit einer anorganischen Verbindung mit hohem spezifischen Gewicht und einer bioadhäsiven Verbindung kombiniert wird. Die Darreichungsform soll ein spezifisches Gewicht von mindestens 1.5 g/cm³ und vorzugsweise 2.0 bis 3 g/cm³ aufweisen.

Es wurde nun gefunden, dass Titanoxid Omeprazol wirksam gegen den Einfluss des Magensafts schützt. Dabei genügt es, Titanoxid erfindungsgemäss mit Omeprazol zu mischen. Eine weitere Komponente, wie z.B. eine bioadhäsive Verbindung, ist hierzu nicht nötig. So hat sich gezeigt, dass sich durch die Zugabe von TiO₂ in den Kern und gegebenenfalls den magensaftresistenten Überzug die Lagerstabilität der erfindungsgemässen Omeprazolformulierung gegenüber Formulierungen aus dem Stand der Technik stark verbessert, und dass dadurch die Verwendung einer als Barriere dienenden separaten Zwischenschicht vermieden werden kann.

Deshalb wird die vorgängig gestellte Aufgabe dadurch gelöst, dass eine pharmazeutische Pelletformulierung mit einem Kern enthaltend Omeprazol in Form seiner freien Base als Wirkstoff und mit einem magensaftresistenten Überzug zur Verfügung gestellt wird, in welcher der Kem und gegebenenfalls der Überzug TiO₂ und weitere Hilfsstoffe enthalten. Als Hilfsstoffe für die erfindungsgemässe Pelletformulierung kommen z.B. Bindemittel, Sedimentationsverzögerer und pH-Korrigentien, in Frage.

Der Wirkstoff Omeprazol wird in Form seiner freien Base verwendet. Entweder wird er als Starterkerne in Form von groben Omeprazolkristallen, vorzugsweise im Korngrössenbereich von 0,2-0,5 mm oder 0,4-1 mm, vorgelegt, oder feinkristallines (z.B. <50 Mikrometer resp. <10 Mikrometer) oder grobkristallines (z.B. <250 Mikrometer) Omeprazol wird in einer Suspension mit Hilfsstoffen auf Starterkerne, z.B. aus Zucker (Saccharose), welche gegebenenfalls Zusätze, wie Natriumcarboxymethylstärke, Polyvinylpyrrolidon, Gelatine oder andere dem Fachmann bekannte Verbindungen enthalten, aufgetragen.

Die Suspension oder Lösung, welche auf die Starterkerne aufgetragen wird, enthält in Wasser oder einem Gemisch aus Wasser und einem oder mehreren üblichen organischen Lösungsmitteln oder in einem organischen Lösungsmittel Omeprazol und Hilfsstoffe, insbesondere mindestens ein Bindemittel, mindestens einen Sedimentationsverzögerer, pH-Korrigentien und gegebenenfalls mindestens einen Farbstoff und/oder Farbpigment und/oder eine Verlackung, Gleitmittel/ Antiklebmittel und Suspensionsstabilisatoren/ Verdicker und als Stabilisator zur Verbesserung der Lagerfähigkeit TiO₂.

Als Bindemittel kommen z.B. Natriumcarboxymethylstärke, Polyvinylpyrrolidon, Gelatine, Hydroxypropylmethylcellulose, Hydroxyethylcellulose, Xanthan, Carrageenan-Produkte, Polyvinylacetat, Natriumcarboxymethylcellulose, Ethylcellulose, Stärkekleister oder verflüssigte Wachse einzeln oder in Kombination miteinander in Frage.

Als Sedimentationsverzögerer kommen z.B. hochdisperses Siliciumdioxid, Stärkekleister, Schleime, wie z.B. Tragant, Gummen, z.B. arabischer Gummi, Xanthane, Alginate oder Carrageenan-Produkte allein oder in Kombination miteinander in Frage.

Als pH-Korrigentien kommen z.B. Natriumhydroxid, Salzsäure, Methylglucamin oder Puffersubstanzen, wie z.B. Natriumdihydrogenphosphat oder Dinatriumhydrogenphosphat, in Frage.

Als Farbstoffe oder Farbpigmente resp. Verlackungen kann man z.B. Eisenoxide, Erythrosin, Gelborange S, Tartrazin oder Indigotin verwenden.

Als Gleitmittel/Antiklebmittel kommt insbesondere Talkum und als Suspensionsstabilisator/Verdicker hochdisperses Siliciumdioxid in Frage.

Der Kern einer erfindungsgemässen pharmazeutischen Formulierung enthält Omeprazol in einer Menge von 20-70 Gew.-%, vorzugsweise 30-50 Gew.-%, und Hilfsstoffe und TiO₂ in einer Menge von 80-30 Gew.-%, vorzugsweise 70-50 Gew.-%, jeweils bezogen auf den Kern des Pellets und unter Abzug des Gewichts der Starterkerne.

TiO₂ liegt dabei etwa in einer Menge von 5-40 Gew-%, vorzugsweise 10-30 Gew.-%, bezogen auf Omeprazol, vor.

Die Omeprazol und TiO₂ enthaltende Suspension I kann zusammen mit der gegebenenfalls TiO₂ enthaltenden Suspension II mit einem Gradientensprühverfahren auf die Starterkerne aufgetragen werden. Begonnen wird dieses Verfahren durch Auftragen der Suspension I auf die Starterkerne, worauf man den hohen Anteil an Suspension I kontinuierlich oder diskontinuierlich z.B. durch Verdünnen der Suspension I mit Suspension II soweit vermindert, dass die Suspension resp. Lösung am Ende dieses Verfahrensschrittes praktisch kein Omeprazol mehr enthält.

Nach dem Auftragen der Suspensionen I und II werden die Pellets in der Beschichtungsanlage oder ausserhalb davon getrocknet. Die Trocknung kann durch Einwirken von Gas, direktem Wärmekontakt, Mikrowellen oder Infrarotstrahlung mit oder ohne Vakuum erfolgen.

Nach Trocknen der Pellets wird ein magensaftresistenter Lack aufgetragen, um die Zersetzung der Pellets im Magensaft zu verhindern. Der Lack wird z.B. in Form einer Suspension in Wasser oder einem Gemisch aus Wasser und organischem Lösungsmittel aufgebracht. Er enthält Filmbildner und gegebenenfalls Bindemittel, Sedimentationsverzögerer, pH-Korrigentien und gegebenenfalls Weichmacher und/oder Farbstoffe, Farbpigmente resp. Verlackungen.

Alternativ kann das Verfahren auch so durchgeführt werden, dass die Omeprazol und TiO₂ enthaltende Suspension I auf die Starterkerne aufgetragen und getrocknet wird, und erst danach Suspension II vorzugsweise mittels eines Gradientensprühverfahrens in Kombination mit dem magensaftresistenten Lack auf die Pellets aufgetragen wird.

Wird der magensaftresistente Lack in Kombination mit TiO₂ aufgetragen, liegt die verwendete Menge an TiO₂ dabei etwa im Bereich von 5-30 Gew-%, vorzugsweise etwa 10-20 Gew.-%, bezogen auf Omeprazol, vor.

Falls TiO₂ auch im Überzug verwendet wird, beläuft sich die Gesamtmenge an TiO₂ im Kern und im Überzug auf etwa 10-40 Gew.-%, vorzugsweise etwa 25-35 Gew.-%, bezogen auf Omeprazol.

Als Filmbildner kommen z.B. Ethylcellulose, Celluloseacetatphthalat, Poly(methacrylsäure, methylmethacrylate) in Frage.

Als Bindemittel eignen sich z.B. Natriumcarboxymethylstärke, Polyvinylpyrrolidon, Gelatine, Hydroxypropylmethylcellulose, Hydroxyethylcellulose, Xanthan, Carrageenan-Produkte, Polyvinylacetat, Natriumcarboxymethylcellulose, Ethylcellulose, Stärkekleister oder verflüssigte Wachse einzeln oder in Kombination miteinander.

Als Sedimentationsverzögerer eignen sich z.B. hochdisperses Siliciumdioxid, Stärkekleister, Schleime, wie z.B. Tragant, Gummen, z.B. arabischer Gummi, Xanthane, Alginate oder Carrageenan-Produkte allein oder in Kombination miteinander.

Als pH-Korrigentien kommen z.B. Natriumhydroxid, Methylglucamin, Salzsäure oder Puffersubstanzen, wie z.B.

Natriumdihydrogenphosphat oder Dinatriumhydrogenphosphat, in Frage.

Als Weichmacher eignen sich z.B. Citronensäureester, wie z.B. Triethylcitrat, Glycerolderivate, wie z.B. Glyceroltriacetat, Salze langkettiger Fettsäuren, wie z.B. Magnesiumstearat, Polywachse, wie z.B. Polyethylenglykol, und/ oder Phthalate, wie z.B. Dibutylphthalat.

Als Farbstoffe oder Farbpigmente resp. Verlackungen kann man z.B. Eisenoxide, Erythrosin, Gelborange S, Tartrazin oder Indigotin verwenden.

Die verwendete Lackmenge beträgt etwa 15-80 Gew.-% des Kerns (inkl. Starterkerne).

Die Herstellung der mit einem magensaftresistenten Film überzogenen Pellets erfolgt z.B. im Kesselverfahren, Trommelcoaterverfahren oder vorzugsweise im Wirbelschichtverfahren (z.B. mittels Hüttlin Kugelcoater, Aeromatic, Glatt o.ä.).

Beim Kesselverfahren werden die Starterkerne im Kessel, z.B. Dragierkessel mit zwiebelförmigem Querschnitt, durch Wandbeheizung oder direkt eingeblasenes warmes Gas, z.B. durch ein Tauchrohr oder Tauchschwert oder anderes Rohr, erwärmt, und dann bei rotierendem Kessel und entsprechender laufender Erwärmung mit den Suspensionen resp. Lösungen besprüht.

Beim Trommelcoaterverfahren wird analog zum Kesselverfahren vorgegangen mit der Ausnahme, dass die Starterkerne in eine perforierte Trommel eingefüllt und dort erwärmt und besprüht werden.

Beim Wirbelschichtverfahren werden die Starterkerne in ∈iner Wirbelschichtanlage z.B. des Typs Aeromatic, Glatt, Kugelcoater Hüttlin o.ä. vorgelegt, mit der Luft oder dem Gas des Wirbelbetts erwärmt und anschliessend bei weiterer Verwirbelung mit den Suspensionen resp. Lösungen besprüht. Im Aufsprühverfahren befindet sich die Düse oder die Düsen oberhalb des wirbelnden Materials, im Unterbettverfahren ist die Düse unterhalb des Wirbelbetts angeordnet. Beim Gleichstromverfahren sprüht die Düse in Durchströmungsrichtung des Gases, beim Gegenstromverfahren umgekehrt.

Nach Trocknung in der Beschichtungsanlage oder in einer separaten Trocknungsanlage wird der magensaftresistente Lack allein oder in Kombination mit der TiO₂ enthaltenden Suspension in einer Anlage, wie oben beschrieben, aufgesprüht, die Pellets mit Überzug sodann getrocknet und die zu kleinen resp. zu grossen Teile durch Sieben oder Windsichten von den Pellets geeigneter Grösse entfernt.

Die Erfindung wird im folgenden anhand der Beispiele näher erläutert.

### Beispiel 1

In einem Wirbelschichtgerät (Aeromatic) wird bei warmer Zuluft 700 g Nonpareilles mit einer Suspension I besprüht, die 37,5 g Hydroxypropylmethylcellulose, 15 g Titandioxid, 18,75 g Dinatriumhydrogenphosphat, 3,75 g hochdisperses Siliciumdioxid und 100 g Omeprazol in 375 g Wasser enthält. Während des Besprühens der Nonpareilles mit der Suspension I wird allmählich eine Suspension II aus 75 g Hydroxypropylmethylcellulose, 11,25 g hochdispersem Siliciumdioxid 18,75 g Dinatriumhydrogenphosphat und 15 g Titandioxid in 1125 g Wasser in die Suspension I hineingegeben und mit dem Besprühen solange fortgefahren, bis die Suspensionen I und II vollständig aufgebracht sind. Die Pellets werden dann mit warmer Luft getrocknet und darauf ein magensaftresistenter Lack aus 150 g Poly(methacrylsäure, methylmethacrylat) , 20 g Triethylcitrat, 15 g Talkum und 10 g Titandioxid in 1000 g Wasser aufgetragen. Die so entstandenen Pellets werden sodann bei 48°C im Wirbelschichtbett bis zur Gleichgewichtsfeuchte getrocknet und dann auf übliche Art in Kapseln abgefüllt.

### Beispiel 2

In einem Wirbelschichtgerät werden bei warmer Zuluft 420 g Zuckerpellets (Nonpareilles) mit einer Suspension I besprüht, die 66 g Omeprazol, 37,5 g Methylhydroxypropylcellulose, 22,5 g Dinatriumhydrogenphosphat, 4,5 g hochdisperses Siliciumdioxid und 13,5 g Titandioxid in Wasser enthält. Nach Beendigung des Auftragens wird getrocknet.

Die getrockneten Pellets werden darauf im obigen WirbeLschichtgerät mit einer Suspension II aus 25 g Methylhydroxypropylcellulose, 8,8 g Titandioxid, 7,5 g hochdispersem Siliciumdioxid und Salzsäure zur pH-Einstellung auf pH 4,0 in 450 g gereinigtem Wasser beschichtet, wobei langsam und unter Rühren eine Suspension III aus 250 g Eudragit L30D und 10 g Triethylcitrat in 250 g gereinigtem Wasser der Suspension II zugemischt wird. Nach erfolgtem Auftrag wird getrocknet und auf übliche Art in Kapseln abgefüllt.

### Beispiel 3

In einem Wirbelschichtgerät werden bei warmer Zuluft 315 g Zuckerpellets (Nonpareilles) mit einer Suspension I besprüht, die 45 g Omeprazol, 33,8 g Methylhydroxypropylcellulose, 9 g Methylglucamin, 4,5 g Titandioxid und 2 g hochdisperses Siliciumdioxid in 360 g gereinigtem Wasser enthält. Nach Beendigung des Auftragens wird getrocknet. Die Pellets werden anschliessend gemäss Beispiel 2 weiterverarbeitet.

### Beispiel 4

Die Stabilität von nach Beispiel 1 hergestellten Omeprazolpellets wurde mit analogen auf dem Markt erhältlichen Omeprazolpellets der Marken ANTRA und SOFEXOL verglichen.

Die weissen Pellets waren jeweils in Hartgelatinekapseln und diese in braune PE-Flaschen abgefüllt. Die Präparate wurden während 6 Monaten bei Temperaturen von 21-25°C (Raumtemperatur) , 31°C (Trockenschrank), 41°C (Trockenschrank) und 40°C (Trockenschrank; relative Feuchte 75%) aufbewahrt und auf ihre Stabilität geprüft.

Es konnte dabei festgestellt werden, dass die 3 Produkte bei Lagerungstemperaturen von 21-25°C, 31°C und 41°C in ihrem Omeprazolgehalt etwa konstant blieben, wobei beim Produkt ANTRA nach 6-monatiger Lagerung bereits bei Raumtemperatur und bei 31°C leichte gelbliche Verfärbugen der Pellets und bei SOFEXOL nach 6-monatiger Lagerung bei einer Temperatur von 31°C gelb-braune Verfärbungen der Pellets festgestellt wurden, wohingegen Verfärbungen beim erfindungsgemässen Produkt bei 6-monatiger Lagerung erst bei 41°C auftraten.

Signifikante Unterschiede im Omeprazolgehalt wurden aber einerseits zwischen den erfindungsgemässen Pellets und andererseits ANTRA resp. SOFEXOL bei einer Lagerungstemperatur von 40°C und relativer Feuchte von 75% festgestellt. Die Daten sind zum besseren Verständnis in der Fig. 1 aufgezeichnet. Gemessen wurde dabei jeweils der Omeprazolgehalt einer Kapsel. Man kann daraus den Schluss ziehen, dass das erfindungsgemässe Produkt wesentlich stabiler als die beiden Referenzprodukte ist.

## Patentansprüche

1. Pharmazeutische Pelletformulierung mit einem Kern enthaltend Omeprazol in Form seiner freien Base als Wirkstoff und einen magensaftresistenten Überzug, wobei der Kern und gegebenenfalls der Überzug TiO₂ und weitere Hilfsstoffe enthalten.

2. Pharmazeutische Formulierung nach Patentanspruch 1, **dadurch gekennzeichnet**, dass als Hilfsstoffe Bindemittel, Sedimentationsverzögerer und pH-Korrigentien verwendet werden.

3. Pharmazeutische Formulierung nach Patentanspruch 1 oder 2, **dadurch gekennzeichnet**, dass TiO₂ in einer Menge von 5-40 Gew-%, vorzugsweise etwa 10-30 Gew.-%, bezogen auf Omeprazol, im Kern vorliegt.

4. Pharmazeutische Formulierung nach einem der Patentansprüche 1-3, **dadurch gekennzeichnet**, dass TiO₂ in einer Menge von 10-40 Gew-%, vorzugsweise etwa 25-35 Gew.-%, bezogen auf Omeprazol, im Kern und Überzug vorliegt.

5. Pharmazeutische Formulierung nach einem der Patentansprüche 1-4, **dadurch gekennzeichnet**, dass Omeprazol in einer Menge von 20-70 Gew.-%, und Hilfsstoffe und TiO₂ in einer Menge von 80-30 Gew.-%, jeweils bezogen auf das Gewicht des Kerns ohne Starterkerne, im Kern vorliegen.

6. Pharmazeutische Formulierung nach einem der Patentansprüche 1-5, **dadurch gekennzeichnet**, dass Omeprazol in einer Menge von 30-50 Gew.-%, und Hilfsstoffe und TiO₂ in einer Menge von 70-50 Gew.-%, jeweils bezogen auf das Gewicht des Kerns ohne Starterkerne, im Kern vorliegen.

7. Pharmazeutische Formulierung nach einem der Patentansprüche 1-6, **dadurch gekennzeichnet**, dass der Überzug 15-80 Gew.-%, bezogen auf das Gewicht des Kerns inkl. Starterkerne, ausmacht.

8. Pharmazeutische Formulierung nach einem der Patentansprüche 1-7, **dadurch gekennzeichnet**, dass als Bindemittel Hydroxypropylmethylcellulose verwendet wird.

9. Pharmazeutische Formulierung nach einem der Patentansprüche 1-8, **dadurch gekennzeichnet**, dass als Sedimentationsverzögerer hochdisperses Siliciumdioxid verwendet wird.

10. Pharmazeutische Formulierung nach einem der Patentansprüche 1-9, **dadurch gekennzeichnet**, dass als pH-Korrigentien Dinatriumhydrogenphosphat oder Methylglucamin verwendet werden.

11. Pharmazeutische Formulierung nach einem der Patentansprüche 1-10, **dadurch gekennzeichnet**, dass der magensaftresistente Überzug Poly(methacrylsäure, methylmethacrylat), Triethylcitrat, Talkum und gegebenenfalls Titandioxid enthält.

12. Verfahren zur Herstellung einer pharmazeutischen Formulierung nach einem der Patentansprüche 1-11, **dadurch gekennzeichnet**, dass anfänglich eine einen hohen Gehalt an Omeprazol enthaltende Suspension I mit Hilfsstoffen und TiO₂ auf Starterkerne aufgetragen wird, der hohe Omeprazolanteil in der Suspension dann kontinuierlich oder diskontinuierlich durch Verdünnen der aufzusprühenden Suspension mit einer Lösung resp. Suspension II von Hilfsstoffen mit gegebenenfalls TiO₂ soweit vermindert wird, dass das Gemisch an aufzutragenden Suspensionen I und II am Ende dieses Verfahrensschrittes praktisch kein Omeprazol mehr enthält, die so entstandenen Pellets dann getrocknet und auf übliche Art mit einem magensaftresistenten Überzug versehen werden.

13. Verfahren zur Herstellung einer pharmazeutischen Formulierung nach einem der Patentansprüche 1-11, **dadurch gekennzeichnet**, dass eine Omeprazol enthaltende Suspension I mit Hilfsstoffen und TiO₂ auf Starterkerne aufgetragen wird, die so entstandenen Pellets dann getrocknet werden, und dann eine Suspension II enthaltend TiO₂ zusammen mit einem magensaftresistenten Überzug aufgetragen wird.

## Claims

1. Pharmaceutical pellet formulation with a core containing omeprazole in the form of the free base as the active substance, and an enteric coating, the core and optionally the coating containing TiO₂ and other adjuncts.

2. Pharmaceutical formulation according to Claim 1, **characterized in that** binders, sedimentation retarders and pH correctors are used as adjuncts.

3. Pharmaceutical formulation according to Claim 1 or 2, **characterized in that** TiO₂ is present in the core in an amount of 5 - 40% by weight, preferably about 10 - 30% by weight, based on omeprazole.

4. Pharmaceutical formulation according to one of Claims 1 - 3, **characterized in that** TiO₂ is present in the core and coating in an amount of 10 - 40% by weight, preferably about 25 - 35% by weight, based on omeprazole.

5. Pharmaceutical formulation according to one of Claims 1 - 4, **characterized in that** omeprazole is present in the core in an amount of 20 - 70% by weight and adjuncts and TiO₂ are present in the core in an amount of 80 - 30% by weight, based in each case on the weight of the core without starter cores.

6. Pharmaceutical formulation according to one of Claims 1 - 5, **characterized in that** omeprazole is present in the core in an amount of 30 - 50% by weight and adjuncts and TiO₂ are present in the core in an amount of 70 - 50% by weight, based in each case on the weight of the core without starter cores.

7. Pharmaceutical formulation according to one of Claims 1 - 6, **characterized in that** the coating is in a proportion of 15 - 80% by weight, based on the weight of the core including starter cores.

8. Pharmaceutical formulation according to one of Claims 1 - 7, **characterized in that** hydroxypropyl methyl cellulose is used as a binder.

9. Pharmaceutical formulation according to one of Claims 1 - 8, **characterized in that** highly disperse silicon dioxide is used as a sedimentation retarder.

10. Pharmaceutical formulation according to one of Claims 1 - 9, **characterized in that** disodium hydrogenphosphate or methylglucamine is used as a pH corrector.

11. Pharmaceutical formulation according to one of Claims 1 - 10, **characterized in that** the enteric coating contains poly(methacrylic acid/methyl methacrylate), triethyl citrate, talcum and optionally titanium dioxide.

12. Process for the preparation of a pharmaceutical formulation according to one of Claims 1 - 11, **characterized in that** a suspension I with a high omeprazole content and with adjuncts and TiO₂ is first applied to starter cores, the high proportion of omeprazole in the suspension is then reduced, continuously or batchwise, by dilution of the suspension to be sprayed with a solution or suspension II of adjuncts, optionally with TiO₂, until the mixture of suspensions I and II to be applied contains practically no more omeprazole at the end of this process step, and the resulting pellets are then dried and provided with an enteric coating in conventional manner.

13. Process for the preparation of a pharmaceutical formulation according to one of Claims 1 - 11, **characterized in that** a suspension I containing omeprazole with adjuncts and TiO₂ is applied to starter cores, the resulting pellets are then dried and a suspension II containing TiO₂ together with an enteric coating is then applied.

## Revendications

1. Formulation pharmaceutique de granules avec un noyau contenant de l'oméprazole sous forme de sa base libre en tant que principe actif et un revêtement gastrorésistant, dans laquelle le noyau et éventuellement le revêtement contiennent du TiO₂ et d'autres substances auxiliaires.

2. Formulation pharmaceutique selon la revendication 1, **caractérisée en ce que** des liants, des retardateurs de sédimentation et des correcteurs de pH sont utilisés en tant que substances auxiliaires.

3. Formulation pharmaceutique selon la revendication 1 ou 2, **caractérisée en ce que** TiO₂ est présent dans le noyau en une quantité de 5 à 40% en poids, de préférence d'environ 10 à 30% en poids, par rapport à l'oméprazole.

4. Formulation pharmaceutique selon l'une des revendications 1 à 3, **caractérisée en ce que** TiO₂ est présent dans le noyau et le revêtement en une quantité de 10 à 40% en poids, de préférence d'environ 25 à 35% en poids, par rapport à l'oméprazole.

5. Formulation pharmaceutique selon l'une des revendications 1 à 4, **caractérisée en ce que** l'oméprazole est présent dans le noyau en une quantité de 20 à 70% en poids, et les substances auxiliaires et TiO₂ sont présents dans le noyau en une quantité de 80 à 30% en poids, dans chaque cas par rapport au poids du noyau sans le noyau de départ.

6. Formulation pharmaceutique selon l'une des revendications 1 à 5, **caractérisée en ce que** l'oméprazole est présent dans le noyau en une quantité de 30 à 50% en poids, et les substances auxiliaires et TiO₂ sont présents dans le noyau en une quantité de 70 à 50% en poids, dans chaque cas par rapport au poids du noyau sans le noyau de départ.

7. Formulation pharmaceutique selon l'une des revendications 1 à 6, **caractérisée en ce que** le revêtement représente de 15 à 80% en poids, par rapport au poids du noyau y compris le noyau de départ.

8. Formulation pharmaceutique selon l'une des revendications 1 à 7, **caractérisée en ce que** l'hydroxypropylméthylcellulose est utilisée en tant que liant.

9. Formulation pharmaceutique selon l'une des revendications 1 à 8, **caractérisée en ce que** le dioxyde de silicium hautement dispersé est utilisé en tant que retardateur de sédimentation.

10. Formulation pharmaceutique selon l'une des revendications 1 à 9, **caractérisée en ce que** l'hydrogénophosphate disodique ou la méthylglutamine sont utilisés en tant que correcteurs de pH.

11. Formulation pharmaceutique selon l'une des revendications 1 à 10, **caractérisée en ce que** le revêtement gastrorésistant contient du poly(acide méthacrylique, méthacrylate de méthyle), du citrate de triéthyle, du talc et éventuellement du dioxyde de titane.

12. Procédé de préparation d'une formulation pharmaceutique selon l'une des revendications 1 à 11, **caractérisé en ce que** une suspension I à teneur élevée en oméprazole est appliquée initialement avec les substances auxiliaires et TiO₂ sur les noyaux de départ, la fraction élevée d'oméprazole dans la suspension est ensuite réduite en continu ou en discontinu par dilution de la suspension à pulvériser avec une solution ou une suspension II de substances auxiliaires avec éventuellement TiO₂ à un point tel que le mélange en suspensions I et II à appliquer à la fin de cette étape du procédé ne contienne pratiquement plus d'oméprazole, et les granules ainsi formés sont ensuite séchés et enrobés de la manière habituelle avec un revêtement gastrorésistant.

13. Procédé de préparation d'une formulation pharmaceutique selon l'une des revendications 1 à 11, **caractérisé en ce que** une suspension I contenant l'oméprazole est appliquée avec les substances auxiliaires et TiO₂ sur les noyaux de départ, les granules ainsi formés sont ensuite séchés et une suspension II contenant TiO₂ ensemble avec un revêtement gastrorésistant y est ensuite appliquée.
